# EUROPEAN PATENT APPLICATION

(11) **EP 4 480 512 A1**
(43) Date of publication of application: **25.12.2024**
(21) Application number: 23180826.2
(22) Date of filing: 22.06.2023
(51) Int. Cl.: A61M 1/06, A61M 39/10

(54) **CONNECTOR FOR A MILK EXPRESSION DEVICE**

(71) Applicant: Koninklijke Philips N.V., 5656 AG Eindhoven (NL)
(72) Inventor: CARTEI, Mirko, Eindhoven (NL); LUI, Kwan Fai, Eindhoven (NL)
(74) Representative: Philips Intellectual Property & Standards

(57) **Abstract**

A connector (1) for use in an expression kit (35) for expressing milk from a mammalian breast,
wherein the connector (1) is configured to be coupled to a source of negative pressure (105),
characterized in that, the connector (1) is configured to be coupled to the expression kit (35) in an orientation selected from a plurality of discrete orientations.

## Description

### FIELD OF THE INVENTION

The invention relates to a connector for use in an expression kit for expressing milk from a mammalian breast, the connector being configured to be coupled to a source of negative pressure. The invention also relates to an expression kit, and an expression system, both for use with the connector.

### BACKGROUND OF THE INVENTION

Such a connector, expression kit, and expression system are known from, for instance, WO2021191637A1.

WO2021191637A1 discloses a breast pump system for expressing milk from a human female breast, the system comprising at least one wearable milk expression kit connected via a tube to a combined external air pump and control unit. Each milk expression kit comprises: (a) a breast shield made up of a breast flange and a nipple tunnel; (b) a flexible diaphragm that is configured to prevent milk from reaching the external air pump; (c) an outer shell that is configured to removably attach to the breast shield, such that, when attached, the breast shield and outer shell form a vessel for collecting milk; and (d) a diaphragm cap that is configured to be secured over the diaphragm, forms part of the front face of the outer shell, and includes a port connected to the tube. From the port, the tube is redirected to pass through a passageway located at the center of the diaphragm cap. This is done to make the milk expression kit fit inside a user's bra. WO2021191637A1 also discloses that, in the terms used in WO2021191637A1, the diaphragm cap is omnidirectional and can be rotated against the outer shell to adjust the position of the port on the outer shell and hence the position and direction of the tube.

It is a drawback of the breast pump system disclosed in WO2021191637A1 that the mechanism to allow the diaphragm cap to rotate against the outer shell is relatively expensive and made of several components. The mechanism should also not allow negative pressure to escape to the environment. It is also a drawback that the diaphragm cap may not maintain its desired orientation relative to the outer housing, for instance because a user may be moving around while using the breast pump system causing the diaphragm cap to rotate against the outer shell. It is also a drawback that redirecting the tube as disclosed in WO2021191637A1 may introduce a kink in the tube, which would negatively impact the fluidic coupling between the external air pump and the expression kit.

### SUMMARY OF THE INVENTION

It is an object of the invention to provide an expression system that is improved compared to the known breast pump systems.

According to the invention, this object is realised in that the connector or the expression kit or both are configured such that the connector and the expression kit can be coupled to each other such that the connector can have an orientation selected from a plurality of discrete orientations relative to a housing of the expression kit.

The invention is based on the insight that the known breast pump system can be simplified if the connector can assume a plurality of discrete positions relative to the housing of the expression kit instead of being omnidirectional as the diaphragm cap in WO2021191637A1.

According to an example, the connector comprises a connector shape configured to provide discrete orientations in which the connector and the expression kit can be coupled to each other.

This example has the additional benefit that it provides a convenient way to enable the plurality of discrete positions. Having the connector comprise a connector shape and the expression kit comprising a corresponding kit shape allows a plug-and-socket approach to providing the plurality of discrete positions. For instance, the connector shape comprises the shape of a part of the connector that is inserted into an inner volume of the expression kit. For instance, the connector shape comprises the shape of a part of the connector that completes an outer contour of the expression kit, i.e that is flush with the housing of the expression kit once the connector and the expression kit have been coupled to each other. As said, the connector shape may be a shape of the connector or of a part of the connector. The term 'connector shape' not only includes the shape of a body comprised in a connector, but may alternatively or additionally also include the arrangement relative to each other of, for instance, connecting interfaces configured to couple a connector and an expression kit to each other. For instance, two protrusions on opposite sides of a circular circumference of a disk-shaped connector can cooperate with two matching indentations in a receiver (for receiving the connector) comprised in an expression kit. Although the connector is disk-shaped, i.e. round in a plane, and as such can be rotated over any angle in this plane, the provision of discrete elements provides for discrete orientations of the connector relative to the expression kit. Similarly, three protrusions around the circular circumference cooperating with matching indentations on an expression kit would provide three discrete orientations for the connector. Such a set of three protrusions will define a triangle between them, resulting in a triangular connector shape. If the three protrusions are evenly spaced, along the circular circumference, they define an equilateral triangle. A pair of protrusions (for instance on opposite sides of a circumference of a connector) may also cooperate with multiple pairs of matching indentations (pairs being defined by indentations on opposite sides of the circumference of an opening matching the connector) resulting in more than two possible discrete orientations for the connector. Of course, it is not essential that protrusions are on the connector and indentations are on an expression kit. Protrusions may be used on the expression kit and matching indentations may be used on a connector. Moreover, matching shapes or elements other than protrusions and indentations may be used, as long as they enable a connector and an expression kit to be coupled to each other (if they would not, they would not be matching; for instance, flexible finger-like elements may be used that can extend from a connector or expression kit into matching openings in an expression kit or connector respectively).

According to an example, the connector shape comprises a non-circular shape.

This example has the additional benefit that having a non-circular shape is a convenient way to provide the connector shape. For instance, the non-circular connector shape is ellipse-shaped. For instance, the non-circular connector shape is polygonal. For instance, the polygonal connector shape is triangular, rectangular, square, rhomboid, pentagonal, or hexagonal. The non-circular shape may be the shape of a body comprised in a connector. For instance, the connector comprises a hexagonal body to be inserted into a receiver comprised in a corresponding expression kit, wherein the receiver has a kit shape matching the connector shape. The non-circular shape may be the shape of connecting interfaces comprised in a connector. For instance, five protrusions evenly spaced along a circular circumference of a disk-shaped connector would define a pentagon between them.

According to an example, the connector comprises a wall portion comprising a port for coupling the connector to a tube for communicating negative pressure to the connector, wherein the wall portion defines a part of an outer contour of an expression kit when the connector and expression kit are coupled to each other, wherein the port defines a direction, and wherein said direction is not perpendicular to the wall portion.

The wall portion may comprise an opening and the port is accessible through the opening. For instance, the port may be in the wall portion. For instance, the port may be extending from the wall portion. This example has the additional benefit that it is not required to have a tube connected to the port make a 90° turn in order to fit inside a shape that fits inside a bra. WO2021191637A1 requires the tube making such a 90° turn.

This example has the additional benefit that changing the orientation of the wall portion will change the orientation of the port. This is especially true for changes in the orientation of the wall portion along an axis perpendicular to the wall portion. This example provides an easy way to change the direction of a tube coupled to the port. If the port were perpendicular to the wall portion, rotating the wall portion around an axis perpendicular to the wall portion would not change the orientation of the port (unless the wall portion is curved and the port is not located on the axis of rotation, because then rotation will change both the position and spatial orientation of the port; therefore, a connector with a curved wall portion and a port perpendicular to the curved wall portion may be used to allow a user to change the position, the orientation, or the position and orientation of a port relative to an expression it).

According to an example, the connector is configured such that changing the orientation of the connector in an expression kit from one orientation to a different orientation defines an axis of rotation and that the port is not positioned on this axis.

This example has the benefit that changing the orientation of the connector in an expression kit will change the position of the port relative to the expression kit. This helps in managing a tube connected to the port. For instance, if the port is positioned along a side of the wall portion, such as when the port is positioned at or near the halfway point of a side, or if the port is positioned at a corner of the wall portion (for instance at a corner of a triangular, square, rhomboid, pentagonal, or hexagonal wall portion), changing the orientation of the connector relative to its expression kit will change to position of the port relative to the expression kit. If the port defines a direction that is not perpendicular to the outer contour, changing the orientation of the wall portion will not only change the position of the port, but also the orientation of the port. This again helps a user to manage tubing.

According to an example, the connector consists of a single piece.

This example has the additional benefit that it is easy to manufacture. It does not require assembly.

The object of the invention is also realized by an expression kit comprising the above connector.

According to an example, the expression kit comprises a receiver for receiving the connector, wherein the receiver comprises a kit shape matching the connector shape.

This example has the additional benefit that it provides a convenient way to enable the plurality of discrete positions. Having the connector have a connector shape and the expression kit having a corresponding kit shape allows a plug-and-socket approach to providing the plurality of discrete positions. For instance, the connector shape comprises the shape of a part of the connector that is inserted into inner volume of the expression kit and the kit shape matches the connector shape. For instance, the connector shape comprises the shape of a part of the connector that is flush with the housing of the expression kit once the connector and the expression kit have been coupled to each other and the kit shape matches the connector shape. For instance, the connector shape relates to the relative positions on the connector of interfaces for coupling the connector to an expression kit and the kit shape relates to the relative positions on the expression kit of interfaces for coupling the expression kit to a connector with a matching connector shape.

According to an example, the expression kit is configured to be coupled to the connector such that, after coupling, the connector is located at a position facing away from a user's breast during use.

This example has the additional benefit that the connector and tube will not interfere with the view that a user has of a surface of the expression kit that faces the user during use. In general, a connector does not obstruct a user's view if the expression kit is configured to be coupled to the connector such that, after coupling, the connector is located at a position that does not lie in a user's field of view when, during use of the expression kit, the user looks at the expression kit. For instance, the connector may be at a position that faces away from a user's torso during use. For instance, the connector may be at a position that faces in the direction of the other breast of a human female user. For instance, the connector may be at a position that faces away from the other breast of a human female user.

According to an example, the expression kit comprising the connector is configured such that, during use, the connector at least partially defines a pumping volume, the pumping volume defining a volume for receiving negative pressure from a source of negative pressure when the connector is coupled to the source of negative pressure.

This example has the additional benefit that a user gets access to one or more parts of the expression kit for cleaning once the connector has been uncoupled from the rest of the expression kit.

According to an example, the expression kit is configured to fit inside a bra.

This example has the additional benefit that it provides a convenient form factor so that the expression kit can be worn in a bra. The form factor is aesthetically pleasing and, by enabling use in a bra, provides a user with a certain degree of mobility, allowing the user to move around while using the expression kit.

The object of the invention is also realized by an expression system comprising the above expression kit.

### BRIEF DESCRIPTION OF THE DRAWINGS

These and other aspects of the invention will be further elucidated and described with reference to the drawing, in which:
Fig. 1 schematically shows examples of connectors according to the invention.
Fig. 2 schematically shows further examples of connectors according to the invention.
Fig. 3 schematically shows examples of expression kits according to the invention as well as a further example of a connector according to the invention.
Fig. 4 schematically shows a further example of an expression kit according to the invention.
Fig. 5 schematically shows an example of an expression system according to the invention.
Fig. 6 schematically shows examples of how to obtain shapes for a receiver for receiving a matching part comprised in a connector or in an expression kit. The drawings also illustrate possible relative arrangements of interfaces for coupling a connector and an expression kit to each other.

### DETAILED DESCRIPTION OF EXAMPLES

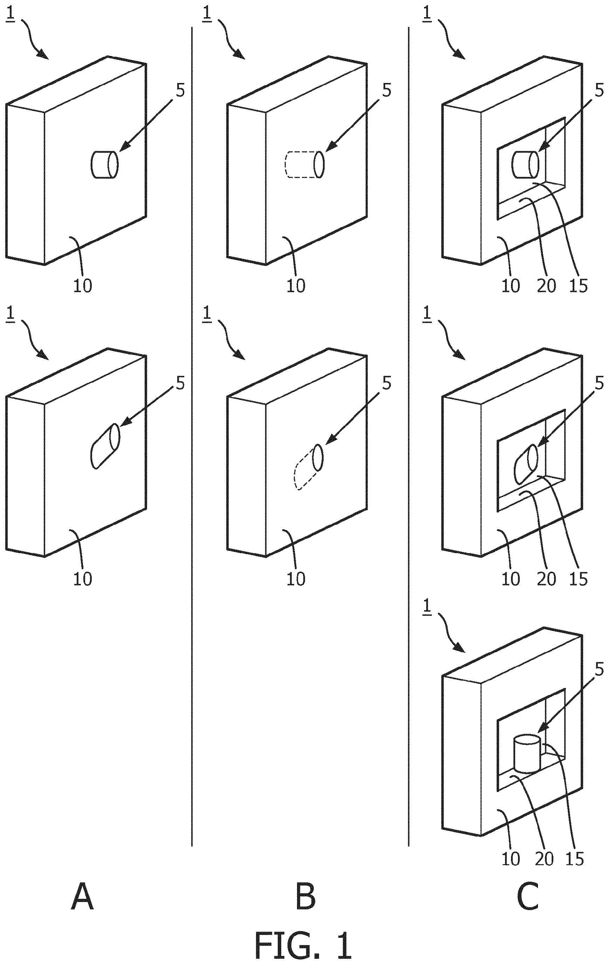

Fig. 1 schematically shows examples of connectors according to the invention. Column A in Fig. 1 schematically shows examples of connectors 1 that comprise ports 5 that protrude from wall portions 10. Ports 5 allow connectors 1 to be coupled to tubing (not shown in Fig. 1) for fluidly connecting connectors 1 to sources of negative pressure (not shown in Fig. 1). Ports 5 shown in the drawings in columns Band C in Fig. 1 serve the same purpose. In the upper of the two drawings in column A, port 5 protrudes perpendicularly from wall portion 10. The direction in which port 5 extends from wall portion 10 is at a 90° angle with the wall portion 10. In the lower of the two drawings in column A, port 5 protrudes non-perpendicularly from wall portion 10. The direction in which port 5 extends from wall portion 10 is at an angle unequal to 90° with wall portion 10. In both drawings in column A, ports 5 comprise plugs.

Column B in Fig. 1 schematically shows examples of connectors 1 that are comprised within the body of the connectors 1, without protruding from wall portions 10. In the upper of the two drawings in column B, port 5 extends perpendicularly relative to the wall portion 10. The direction in which port 5 extends has a 90° angle relative to the wall portion 10. In the lower of the two drawings in column B, port 5 extends non-perpendicularly relative to wall portion 10. The direction in which port 5 extends has an angle unequal to 90° relative to the wall portion 10. In both drawings in column B, ports 5 comprise holes.

Column C in Fig. 1 schematically shows examples of connectors 1 that comprise recesses 15 in wall portions 10, wherein the ports 5 are positioned within the recesses 15. In the examples in column C, recesses 15 are defined by openings in wall portions 10, sidewalls 20, and base surfaces which, in the examples shown, are parallel to wall portions 10. In the drawings in column C, the recesses 15 comprise rectangular openings in wall portions 10. However, in other examples the recesses 15 comprise openings that have other shapes, for instance circular, oval or triangular shapes. In the upper drawing in column C, port 5 extends in a direction that is perpendicular to wall portion 10. The direction in which port 5 extends has a 90° angle relative to the wall portion 10. In the middle drawing in column C, port 5 extends in a direction that is not perpendicular relative to the wall portion 10. In the middle drawing in column C, port 5 port 5 extends non-perpendicularly relative to wall portion 10. The direction in which port 5 extends has an angle unequal to 90° relative to the wall portion 10. In the lower drawing in column C, port 5 extends from a side wall 20. In the example shown, port 5 extends perpendicularly from the side wall 20. It will be clear that in another example a port 5 may extend non-perpendicularly from a side wall 20. In all drawings in column C, ports 5 comprise plugs. However, it will be clear that ports 5 in column C might alternatively comprise holes extending into the base surfaces or the sidewalls 20. Such holes may extend in directions that are either perpendicular or non-perpendicular to the base surfaces or the sidewalls 20. In the drawings in column C, ports 5 do not extend the planes defined by wall portions 10. However, it will be clear that in alternative examples the ports 5 may extend from the recesses 15 beyond the planes defined by wall portions 10.

In Fig. 1 all connectors 1 comprise ports 5 extending from or within bodies having rectangular shapes. In combination with expression kits comprising receivers for receiving such connectors 1, the connectors 1 can be coupled to the expression kits in four discrete orientations. It will be clear that rectangular shapes, including square shapes, are not the only shapes providing such pluralities of discrete orientations. In examples other than those shown in Fig. 1, the bodies of connectors 1 have polygonal shapes, for instance, triangular, rhomboid, pentagonal, or hexagonal shapes.

In Fig. 1 wall portions 10 are flat. However, in examples other than those shown in Fig. 1, wall portions 10 are curved (in the sense that at least a surface this is an out surface during use is curved), for instance to complete curved outer surfaces of expression kits. In such instances, whether or not a port extends perpendicularly or non-perpendicularly relative to a wall portion 10 has to be assessed locally at the position of the port. If a port extends from a base surface of a recess or from a side wall of a recess, whether or not the port extends perpendicularly or non-perpendicularly relative to the base surface or side wall has to be assessed locally at the position of the port.

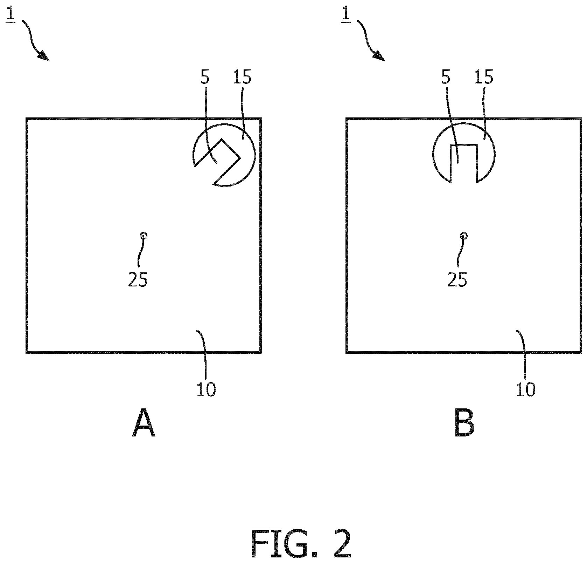

Fig. 2 schematically shows further examples of connectors according to the invention. Both drawings in Fig. 2 schematically show frontal views connectors 1. The connectors 1 comprise ports 5 extending from sidewalls within recesses 15. In both drawings the ports 5 extend from or within bodies having square shapes. So far, the connectors 1 are similar to the example shown in the lower drawing in column C in Fig. 1. However, while the recess 15 in the lower drawing in column C in Fig. 1 has a rectangular opening, the recesses 15 in the drawings in Fig. 2 have circular openings.

A difference between the example shown in the lower drawing in column C in Fig. 1 and the examples shown in Fig. 2 is that in the example in Fig. 1 port 5 is on or near a central axis perpendicular to the wall portion 10. In the example in Fig. 2, ports 5 are positioned off-axis relative to the central axes 25 perpendicular to the wall portions 10. As already explained in relation to the one, the square-shaped bodies of the connectors 1 in Fig. 2 provide four discrete orientations in which the connectors 1 can be received in corresponding receivers comprised in expression kits. The four discrete orientations are obtained by rotating the connectors 1 around the axes 25. Because the positions of ports 5 in Fig. 2 do not lie on the axes 25, rotating the connectors 1 along the axes 25 will change the position of the ports 5 (if the ports 5 extend perpendicularly from or within the bodies of connectors 1) or the position and the orientation of the ports 5 (if the ports 5 extend non-perpendicularly from or within the bodies of connectors 1) relative to expression kits in which the connectors 1 are received.

In drawing A in Fig. 2, port 5 is positioned at a corner of the square-shaped body of the connector 1. Alternatively, port 5 is positioned along a side of the square-shaped body of the connector 1. In the example given in drawing B, port 5 is positioned at the midpoint of one side of the body of the connector 1. As discussed already in relation to Fig. 1, the bodies of the connectors 1 may have polygonal shapes, for instance triangular, rhomboid, pentagonal, or hexagonal shapes. Also with such non-square, polygonal-shaped bodies, ports 5 may be positioned at corners or along sides of such bodies. Ports 5 on bodies having non-polygonal shapes, like oval shapes, may similarly be positioned off a central axis, along a side of the body or at or near an extremity of a minor or major axis.

Following the discussion of Fig. 2, it will be clear that each of the ports 5 discussed in relation to Fig. 1 may be positioned off the central axes of the bodies of the connectors 1 discussed in relation to Fig. 1 if one wants rotation of the bodies along the central axes to change the positions or the positions and orientations of the ports 5 relative to expression kits in which the connectors are received.

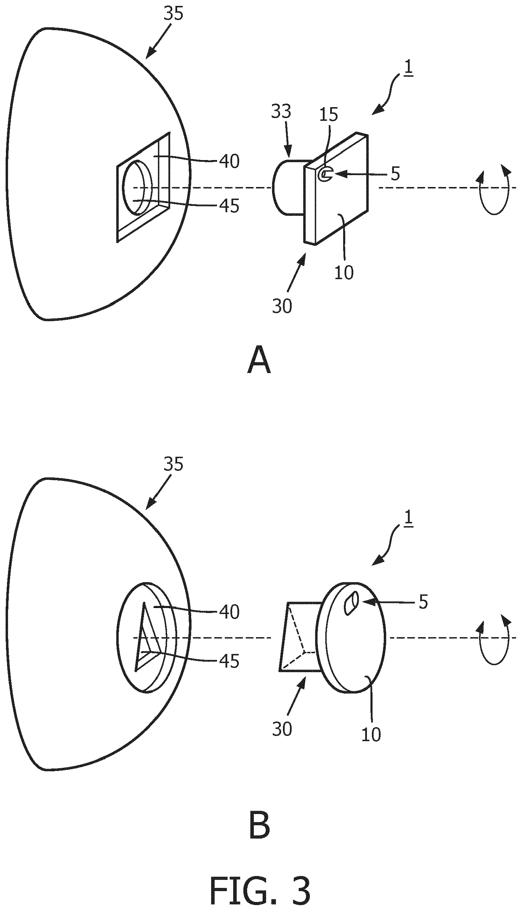

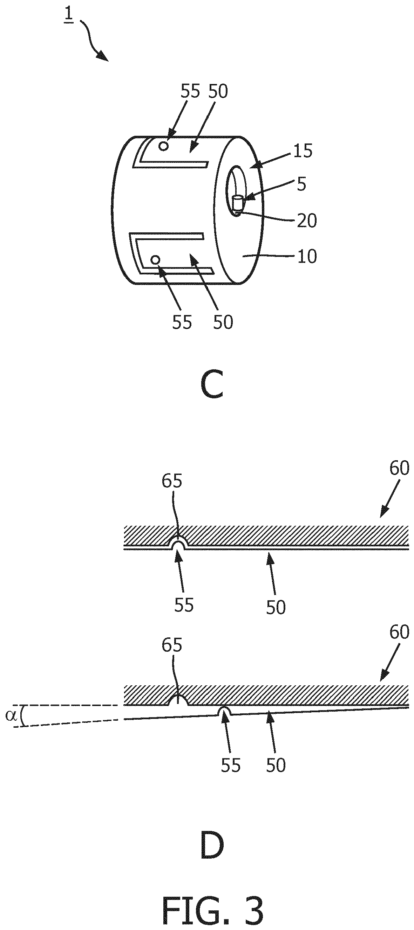

Fig. 3 schematically shows examples of expression kits according to the invention as well as a further example of a connector according to the invention. Drawing A in Fig. 3 schematically shows a connector 1 comprising a part 30 comprising port 5 within recess 15 in a configuration that is similar to the configuration shown in drawing A in Fig. 2. The part 30 comprises a square connector shape. The connector 1 further comprises a cylindrically-shaped part 33. Because of the square connector shape and a receiver 40 with a square kit shape matching the square connector shape on the expression kit 35, connector 1 can be coupled to the expression kit 35 in four different, discrete orientations. The four orientations are obtained by rotating connector 1 as indicated by the double-headed circular arrow. As port 5 is not perpendicular to the wall portion 10, port 5 changes both its position and its orientation as the orientation of connector 1 is changed from one orientation to another. Being able to change the position or the position and orientation of port 5 allows a user to manage the position respectively the position and orientation of tubing that can be coupled to the port 5 to fluidically couple the port 5 to a source of negative pressure. In this way, a user can select a preferred configuration. In the example shown, after coupling of the connector (1) and the expression kit (35), surface 10 is flush with the neighboring outer surface of the expression kit. Obviously, this is not essential.

Drawing B in Fig. 3 schematically shows a connector 1 comprising a circular-shaped part, which comprises port 5 extending non-perpendicularly from wall portion 10. The connector 1 further comprises a part 30 comprising a triangular connector shape. Because of the triangular connector shape and a receiver 45 with a triangular kit shape matching the triangular connector shape on the expression kit 35, connector 1 can be coupled to the expression kit 35 in three different, discrete orientations. The three orientations are obtained by rotating connector 1 as indicated by the double-headed circular arrow. As port 5 is not perpendicular to the wall portion 10, port 5 changes both its position and its orientation as the orientation of connector 1 is changed from one orientation to another. Being able to change the position or the position and orientation of port 5 allows a user to manage the position respectively the position and orientation of tubing that can be coupled to the port 5 to fluidically couple the port 5 to a source of negative pressure. In this way, a user can select a preferred configuration. In the example shown, after coupling of the connector (1) and the expression kit (35), surface 10 is flush with the neighboring outer surface of the expression kit. Obviously, this is not essential.

In Fig. 3 the connectors 1 comprise parts comprising connector shapes that enable pluralities of discrete orientations of connectors 1 relative to expression kits 35 when the connectors 1 and expression kits 35 are coupled to each other. Connector shapes do not only include shapes of whole bodies like the square-shaped part 30 in drawing A in Fig. 3 or the triangular-shaped part 30 in drawing B in Fig. 3, but also the configuration of interfaces comprised in bodies. Suppose, for instance, that the part 30 in drawing A in Fig. 3 would not have a square shape, but a circular shape, either with the same diameter as the circular-shaped part shown in drawing A (in which event the whole connector would look like a cylinder) or with a larger diameter than the cylindrically-shaped part shown in drawing A (in which event the connector would look like two cylinders of different diameters connected in series). In this situation, a plurality of discrete orientations could be enabled, for instance, by having one or more protrusions and/or indentations at predetermined positions along the circumference of either or both cylindrical parts. The one or more protrusions and/or indentations would cooperate with matching features comprised in a corresponding expression kit. The features on the connector and the matching features on the expression kit would form interfaces providing a plurality of discrete orientations for the connector relative to the expression kit when the connector and the expression kit are coupled to each other. The positions of these features relative to each other on a connector or an expression kit would define a connector shape and a kit shape respectively. For example, three evenly spaced protrusions along the circumference of one of the cylindrical parts and three matching indentations in a corresponding expression kit would provide three different, discrete orientations for the connector relative to the expression kit. The protrusions on the connector would define the connector shape (triangle) and the indentations on the expression kit would define the kit shape (triangle). Such an arrangement may require a release mechanism to uncouple the connector and the expression kit if and when needed. Such a release mechanism could comprise the protrusions or the indentations being positioned on flexible elements that can flex to release a protrusion and an indentation from each other. Such flexible elements would also allow a connector and its corresponding expression kit to move relatively to each other when protrusions and indentations are not aligned. For instance, a flexible element comprising a protrusion could be bent in one direction when protrusions and indentations do not align and relax in the opposite direction and snap into place when protrusions and indentations do align. The flexibility could then also provide a biasing force keeping a protrusion in an indentation as long as the biasing force would not be overcome by an external force (for instance a force exerted by a user when she wants to uncouple the connector and the expression kit).

Drawing C in Fig. 3 schematically shows a connector as described in the previous paragraph. Connector 1 as a whole is shaped as a cylinder. Port 5 is positioned off the central, longitudinal axis of the cylinder. Port 5 is comprised in a recess 15. In this example, port 5 extends from a side wall 20 of the recess 15, which is comprised in wall portion 10. The connector 1 comprises a plurality of flexible elements 50, shaped here as rectangular components that are connected to the rest of connector 1 at only one of their sides. Towards their free ends, flexible elements 50 comprise protrusions 55. Such protrusion, or alternatively indentations, are not essential. The flexible elements (with or without protrusion or indentations) may also be configured with a bias to extent outward from the rest of the connector.

Drawing D in Fig. 3 schematically shows cross sections of the interface between a part 60 comprised in an expression kit 35 and a flexible element 50 comprised in a connector 1 as shown in drawing C in Fig. 3. In the upper part in drawing D, protrusion 55 extends into indentation 65 when connector 1 and expression kit 35 are coupled to each other. In the lower part in drawing D, protrusion 55 and indentation 65 are not aligned and protrusion 55 touches the flat wall of part 60. Consequently, flexible element 50 is bent as illustrated by the angle α subtended by the dashed lines. This situation occurs when connector 1 is put into or is removed from expression kit 1.

In drawings C and D, the flexible elements 50 extend in the longitudinal direction of connector 1. However, it is also conceivable to have flexible elements like flexible elements 50 that extend circumferentially. Such flexible elements may be biased to have their free ends extend outwards from the circumference of a connector when the connector is not coupled to a corresponding expression kit. The extending free ends would form a sawtooth pattern, similar to the shape of a toothed blade for a power saw. If the connector is coupled to its corresponding expression kit, the flexible elements would then interface with indentations in a surface of the expression kit that faces the connector when the connector is inserted into the expression kit. The indentations could have a sawtooth pattern and match the free ends extending from the connector. Here it says `could have a sawtooth pattern', because although indentations with sawtooth shape would nicely fit with flexible elements that also form a sawtooth pattern, what matters is that the indentations and the flexible elements cooperate with each other. An exact fit may be nice, but is often not essential. Rotating the connector inside the expression kit on one direction would alternatingly fix and free the connector. During rotation of the connector in this direction, the connector would be fixed in place if its flexible elements snugly fit into the indentations having a sawtooth shape. If the connector were rotated further (by overcoming the force of the free ends in the indentations) in the direction in which the amplitude of the indentations (i.e. the depth of the indentations) decreases, the free ends of the flexible elements would be forced to move towards the rest of the connector. Once the free ends of the flexible elements would again align with the next indentations, the free ends of the flexible elements would move away from the rest of the connector and again snugly fit into the indentations. The free ends would then be aligned with indentations next to the indentations into which the free ends fitted prior to the further rotation. If the connector were rotated in the opposite direction, the free ends of the flexible elements would hit surfaces at the deeper ends of the indentations having a sawtooth shape (i.e. the free ends would run into surfaces at the wider end a sawtooth-shaped indentations). Thus, further rotation in the latter direction would be blocked.

Indentations and protrusions, for instance the indentations and/or protrusions on flexible elements like the flexible elements discussed above, may be used to keep any connector according to the invention coupled to an expression kit. For example, any of the connectors in Fig. 1 may comprise flexible elements with free ends in two opposing side surfaces, i.e. two of the surfaces perpendicular to wall portion 10. The free ends may comprise one or more protrusion or indentations to cooperate with matching features in a corresponding expression kit. Such flexible elements could extend from their free ends to the opposite ends (at which they are connected to the rest of their connector) in a direction perpendicular to the wall portion 10. The free ends could be arranged to enter an expression kit first when a connector comprising the free ends would be inserted into an expression kit. This arrangement would allow a user to squeeze the free ends towards the rest of the connector for insertion of the connector into an expression kit. Once aligned with matching features (e.g. indentations with sawtooth shapes) in the expression kit, the free ends would relax and snugly fit into the matching features, possibly by extending from the rest of the connector. Pulling on the connector would result in the free ends being forced towards the rest of the connector again, thereby releasing the connector form the expression kit. This lock-release mechanism as such is similar to the mechanism used in BIC^{™} M10 pens to extend and retract the writing tip. However, one difference, is that, contrary to operation of the BIC^{™} M10 pen, one cannot press on a free end of a flexible element of a connector while the connector is in an expression kit. However, a connector may comprise an actuator coupled to the free ends, for instance a press button, to force the free ends to move towards the rest of their connector, thus allowing removal of the connector from its expression kit. Removal of a connector from its expression kit allows cleaning of the connector and of the expression kit or at least a part thereof.

In addition to connectors 1, drawings A and B in Fig. 3 further show expression kits 35. Both expression kits 35 comprise receivers (40, 45) configured for receiving the respective connectors 1, specifically the parts 30 (of the connectors 1) comprising the connector shapes. The receiver 40 in drawing A in Fig. 3 is configured to receive the square-shaped part 30 of the corresponding connector 1. That is why the receiver 40 has a square kit shape. The receiver 45 in drawing B in Fig. 3 is configured to receive the triangular-shaped part 30 of corresponding connector 1. That is why the receiver 45 has a triangular kit shape.

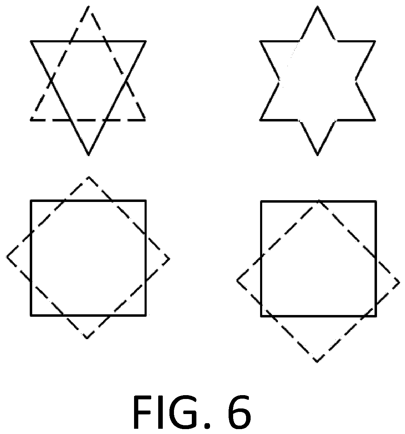

Given a certain symmetry for a connector, for instance the four-fold symmetry of the connector shape of connector 1 in drawing A in Fig. 3, more than four discrete orientations for connector 1 can be obtained using an appropriate receiver on an expression kit. If the receiver 40 in drawing A in Fig. 3 were not square-shaped, but shaped like two concentrical, identically sized squares rotated relative to each other, additional discrete orientations for the connector 1 would become available, without requiring any changes to connector 1. The angle of rotation between the two imaginary squares defining the receiver in this example, might be such that the resulting shape of the receiver is symmetrical. For example, rotating the two imaginary squares relative to each other over an angle of 45° would result in eight, evenly spaced possible discrete orientations for connector 1. Rotation over an angle other than 45° (but not 90°, because then the two imaginary square would coincide resulting in the example already shown in drawing A in Fig. 3), would also provide eight possible discrete orientations for connector 1. However, they would not be evenly spaced around the axis of rotation. It is also not essential that the two squares are concentric. One could also use two identically-sized squares that are overlapping, but not concentric, and rotated relatively to each other. See also Fig. 6 and its description.

The same approach can be applied to the connector 1 in drawing B in Fig. 3. This connector 1 has a three-fold symmetry. More than three possible discrete orientations for the connector 1 can be obtained if the receiver 40 in drawing B in Fig. 3 were not shaped as a triangle, but shaped like two identically sized triangles rotated relative to each other and that possibly are concentrical. A receiver 40 resulting from this approach has the contours of a star of David providing six possible discrete orientations for the connector. However, just as with the imaginary squares, the imaginary triangles can be rotated relative to each other over any angle, as longs as they do not coincide. Six possible discrete orientations would again be obtained for the connector, but they would not be evenly spaced around the axis of rotation. As with the squares discussed above, it is not essential that the two triangles are concentric. One could also use two identically-sized triangles that are overlapping, but not concentric, and rotated relatively to each other. The above approach is illustrated in Fig. 6. The top-left drawing in Fig. 6 schematically shows two concentric, overlapping triangles (one drawn with a solid line, one drawn with a dashed line), defining an outer contour in the form of a star of David. This outer contour is shown in the top right drawing in Fig. 6. The bottom-left drawing in Fig. 6 schematically shows two concentric, overlapping squares (one drawn with a solid line, one drawn with a dashed line). The bottom-right drawing in Fig. 6 schematically shows two non-concentric, but still overlapping squares.

In Fig. 3, the receivers 40 and 45 with the kit shapes are shown as openings, whereas the parts 30 with the connector shapes are shown as protrusions. It will be clear that these arrangements can be reversed with the expression kits comprising protrusions and the connectors comprising openings. With the previous paragraphs in mind, this also means that according to the invention one may have connectors with openings that provide additional discrete orientations. For example, an expression kit might have an elongated rectangular protrusion as a kit shape and the matching connector would have a connector shape in the form of a cross-shaped opening, with each arm of the cross configured to receive the protrusion of the expression kit. The arms of the cross may be perpendicular to each other. In another example, the arms of the cross are at a non-right angle to each other. In this latter example the cross would be skewed. In general, one can have a smaller plug (e.g. an elongated rectangle) and fit it into a larger socket, with the socket being larger in the sense that the shape of the socket results from rotating two copies of the plug relative to each other (i.e. the socket has the shape of a cross or a skewed cross resulting from rotating two identically-sized elongated rectangles relative to each other). The two copies need not be arranged concentrically. Either of the plug and socket may be comprised in the connector with the other of the plug and socket being comprised in the expression kit.

As said earlier, connector shapes and kit shapes are not limited to the shapes of whole bodies, but also include the configurations of interfaces comprised in bodies. Thus, what has been said above about obtaining additional possible orientations also applies to such interfaces. For instance, a connector like connector 1 in drawing C in Fig. 3 could comprise two of the flexible elements 50. These two flexible elements 50 might be arranged on opposite sides of the connector 1. To obtain additional possible, discrete orientations, the corresponding expression kit could have not two, but four elements matching the flexible elements 50. The four matching elements would be arranged in two pairs, with each pair matching the pair of flexible elements 50 on connector 1. In this way, with two flexible elements on the connector 1 one would not have two, but four possible orientations for the connector. This is similar to the approach of having a plug with an elongated rectangular shape (with two opposing smaller or longer sides) and a matching socket shaped as two identical elongated rectangular shapes (that match the elongated rectangular shape of the plug) rotated relative to each other. The socket would then present two pairs of smaller or longer sides. In short, what has been said about obtaining additional orientations applies to both the shapes of whole bodies and to the configurations of interfaces. It also does not matter in which direction interfaces extend. Above, flexible elements 50 have been discussed that extend longitudinally or circumferentially on connector 1 in drawing C in Fig. 3. The above approach to obtain additional possible orientations applies regardless of the direction of extension. For example, one could have two flexible elements extending from a connector and four (i.e. two pairs of) sawtooth-shaped indentations on a corresponding expression kit.

It is also noted that different shapes can provide the same number of symmetries and, hence, possible discrete orientations for a connector according to the invention. For example, a square connector shape offers four-fold symmetry. However, a cross-shaped connector shape would do the same. Similarly, a triangular connector shape like the one shown in drawing B in Fig. 3 provides three-fold symmetry. Three pins arranged along a equilateral triangle would do the same. A connector and a corresponding expression kit, both according to the invention, form a plug-and-socket system, wherein the plug and socket can have any shape as long as they match. To avoid any misunderstanding, the fact that plugs and sockets match includes the approach of creating additional symmetries as discussed above.

Drawings A and B in Fig. 3 show that connectors 1 may comprise both parts that are configured to enable pluralities of discrete orientations (the parts 30 with respectively square and triangular connector shapes) and parts that do not serve this purpose (the circular-shaped parts of the connectors 1). Either type of part may complete an outer surface of the expression kit to which connectors 1 are coupled. Similarly, drawings A and B in Fig. 3 show that expression kits 35 may comprise both receivers (for receiving connectors 1) that are configured to enable pluralities of discrete orientations of the connectors 1 and elements that do not serve this purpose (the circular openings 45).

Drawings A and B in Fig. 3 also show that ports 5 may extend from (drawing B) or within (drawing A) parts having bodies that have different shapes, a square shape in drawing A and a circular shape in drawing B. In drawing A, port 5 extends perpendicularly from a side wall of recess 15 wall portion 10 while being positioned off the central axis of the square-shaped part 30 of connector 1. In drawing B port 5 extends non-perpendicularly from wall portion 10 while being positioned off the central axis of the circular part of connector 1. However, the fact that ports can extend perpendicularly or non-perpendicularly from or within parts having bodies of different shapes has already been discussed in relation to Figs. 1 and 2. The fact that ports can be positioned on or off central axes has been discussed in relation to Fig. 2.

Drawings A and B in Fig. 3 show expression kits 35 without the connectors 1 having been coupled to them. This illustrates that the expression kits 35 and the connectors 1 form so-called 'plug-and-socket' systems in which the expression kits 35 and the connectors 1 cooperate such that the connectors 1 can be coupled to the expression kits 35 in a plurality of discrete orientations of the connectors 1 relative to the expression kits 35.

It will be clear that in order to express milk from a mammalian breast, for instance a human female breast, the expression kits 35 and the connectors 1 need to be combined. For reasons of conciseness, the combination of an expression kit 35 (i.e. an expression kit without a connector) and a connector 1 will be called 'an expression kit comprising a connector'.

Drawings A and B in Fig. 3 show that at least a part of the connectors 1 may serve to complete an outer surface of expression kits 35. After coupling the expression kits 35 and connectors 1 in drawings A and B in Fig. 3, the wall portions 10 of the connectors 1 complete outer surfaces of the expression kits now comprising the connectors. As we mentioned in relation to Fig. 1, the wall portions 10 may be flat or curved.

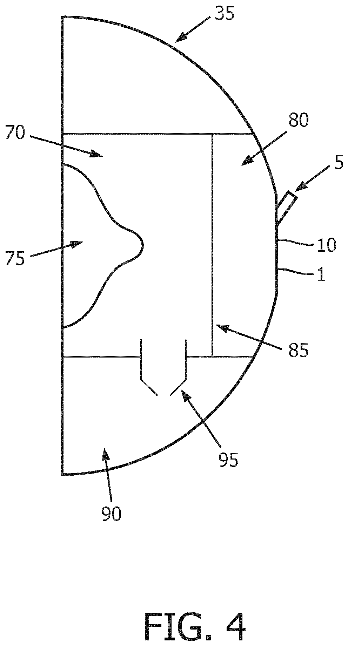

Fig. 4 schematically shows a further example of an expression kit comprising a connector according to the invention. Fig. 4 shows a cross-section along a vertical plane through the expression kit 35 comprising the connector 1. Here, the vertical direction is defined as the up-down direction during normal use of the expression kit, with the user standing or sitting in an upright position. The expression kit 35 comprises the connector 1. In this example, wall portion 10 is flat and completes an otherwise curved outer surface of the expression kit 35. In this example, port 5 is positioned off the central axis of connector 1 and extends non-perpendicularly from wall portion 10. The expression kit 35 comprises a breast volume 70 for receiving a breast 75. The expression kit 35 further comprises a pumping volume 80. Through port 5, the pumping volume 80 can be fluidic coupled to a source of negative pressure (not shown in Fig. 4). The pumping volume 80 is at least partially defined by the connector 1. In Fig. 4, the pumping volume 80 is partially defined by a membrane 85 separating the breast volume 70 from the pumping volume 80. The membrane 85 is flexible. As the pressure in the pumping volume 80 changes, membrane 85 deforms changing the pressure in the breast volume 70. Creating a negative pressure in the breast volume 70 generates suction on the breast 75 which, in turn, facilitates the expression of milk from breast 75.

In the example shown in Fig. 4, the expression kit 35 further comprises a milk volume 90 for collecting expressed milk. The breast volume 70 in the milk volume 90 are connected via a one-way valve 95. One-way valve 95 allows milk to enter the milk volume 90 from the breast volume 70, but prevents milk from flowing from the milk volume 90 back to the breast volume 70.

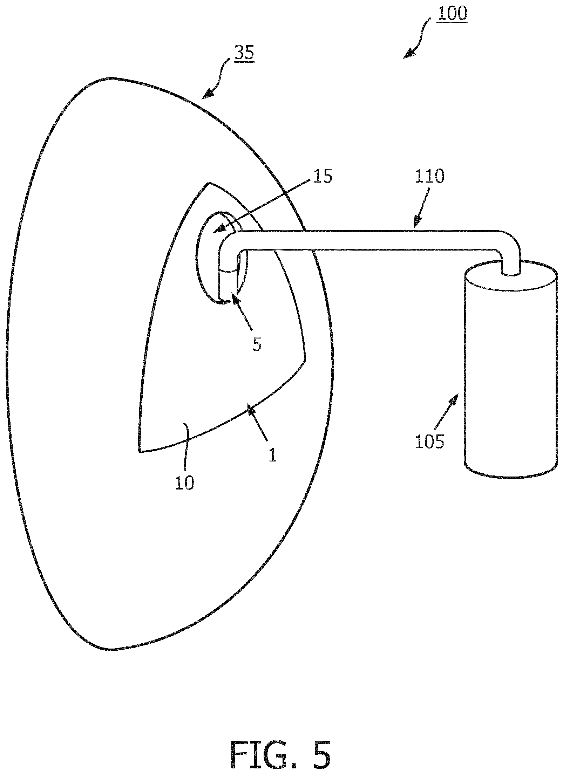

Fig. 5 schematically shows an example of an expression system according to the invention. Fig. 5 shows an expression system 100. The expression system 100 comprises both an expression kit 35 comprising a connector 1 and a source of negative pressure 105. The source of negative pressure 105 may comprise a pump. This pump may be electrically operated or may be manually operated. A manually operated pump may comprise a lever that may be actuated to generate a negative pressure. The source of negative pressure 105 is fluidly coupled to port 5 via tubing 110.

Port 5 is comprised in connector 1. In the example shown, wall portion 10 of connector 1 has the shape of a curved triangle. Wall portion 10 completes the curved outer shape of the expression kit 35 comprising connector 1. Port 5 is positioned at one corner of the curved triangle. As explained earlier, in alternative examples port 5 is positioned along a side of the curved triangle. As also explained earlier, what is a triangular-shaped in Fig. 5 may be another shape in other examples. Moreover, as discussed in relation to drawing B in Fig. 3, the wall portion 10 need not be comprised in the part of connector 1 that is configured to enable a plurality of discrete orientations of connector 1 relative to expression kit 35. In Fig. 5, port 5 is positioned within recess 15. As discussed earlier, in other examples port 5 is not positioned within a recess, but is extending from or within the body of a part of the connector 1.

The expression system 100 in Fig. 5 comprises a single expression kit 35 comprising connector 1. In another example, the expression system comprises two or more expression kits 35, each comprising a connector 1. If the expression system 100 comprises two or more expression kits 35, each comprising a connector 1, one or more sources negative pressure may be use. For example, a single source of negative pressure may be used to generate negative pressure in two expression kits.

In Fig. 5, connector 1 is positioned such that, during milk expression, wall portion 10 faces away from a human female user. This position prevents the connector 1 from obstructing the user's view. If a connector would be positioned in the area indicated by the arrow of reference numeral 35, this connector could obstruct a user's view during milk expression.

By way of example, Fig. 5, but also Figs. 3 and 4, show expression kits 35 (with or without connectors 1) that are configured to fit inside bras. Such exception kits provide additional comfort and mobility compared to more traditional expression kits that comprise funnels for receiving breasts, wherein the funnels are coupled to bottles for collecting expressed milk.

## Claims

1. A connector (1) for use in an expression kit (35) for expressing milk from a mammalian breast, wherein the connector (1) is configured to be coupled to a source of negative pressure (105),
**characterized in that**,
the connector (1) is configured to be coupled to the expression kit (35) in an orientation selected from a plurality of discrete orientations.

2. The connector (1) according to any of claim 1, wherein the connector (1) comprises a connector shape configured to provide discrete orientations in which the connector (1) can be coupled to the expression kit (35).

3. The connector (1) according to claim 2, wherein the connector shape comprises a non-circular shape.

4. The connector (1) according to any of claim 3, wherein the connector shape is chosen from the following group of shapes: triangle, rectangle, square, rhomboid, pentagon, hexagon.

5. The connector (1) according to any of claims 2-4, wherein the connector shape is defined by an interface for coupling the connector (1) and an expression kit (35) to each other.

6. The connector (1) according to any of claims 1-5, wherein connector (1) comprises a wall portion comprising a port (5) for coupling the connector (1) to a tube for communicating negative pressure to the connector (1), wherein the wall portion defines a part of an outer contour of an expression kit when the connector (1) and expression kit are coupled to each other, wherein the port (5) defines a direction, and wherein said direction is not perpendicular to the outer contour.

7. The connector (1) according to any of claims 1-6, wherein the connector (1) is configured such that changing the orientation of the connector (1) in an expression kit from one orientation to a different orientation defines an axis of rotation and that the port (5) is not positioned on this axis.

8. The connector (1) according to any of claims 1-7, wherein the connector (1) consists of a single piece.

9. An expression kit (35) for expressing milk from a mammalian breast, the expression kit (35) being configured for placement on the mammalian breast for expressing milk,
**characterized in that**,
the expression kit (35) is configured to be coupled to the connector (1) according to any of claims 1-8.

10. The expression kit (35) according to claim 9 with reference limited to the connector (1) according to any of claims 2-5, wherein the expression kit (35) comprises a receiver for receiving the connector (1), wherein the receiver comprises a kit shape matching the connector shape.

11. The expression kit (35) of any one of claims 9-10, wherein the expression kit (35) is configured to be coupled to the connector (1) such that, after coupling, the connector is located at a position facing away from a user's breast during use.

12. The expression kit (35) according to any of claims 9-11, wherein the expression kit (35) comprises the connector (1) according to any of claims 1-8.

13. The expression kit (35) according to claim 12, wherein the expression kit (35) comprising the connector (1) is configured such that, during use, the connector (1) at least partially defines a pumping volume (80), the pumping volume (80) defining a volume for receiving negative pressure from a source of negative pressure (105) when the connector (1) is coupled to the source of negative pressure (105).

14. The expression kit (35) according to any one of claims 9-13, wherein the expression kit (35) is configured to fit inside a bra.

15. An expression system (100) for expressing milk from a mammalian breast comprising the expression kit (35) according to any of claims 9-14.
